# EUROPÄISCHE PATENTANMELDUNG

(11) **EP 0 985 684 A2**
(43) Veröffentlichungstag der Anmeldung: **15.03.2000**
(21) Anmeldenummer: 99116771.9
(22) Anmeldetag: 30.08.1999
(51) Int. Cl.: C08F 4/40, C09J 4/00, A61L 24/06, A61K 6/083

(54) **Neue Reaktivsysteme aus polymerisierbaren Monomeren, die Peroxide und stabilisierte Boralkylverbindungen enthalten**

(30) Priorität: 10.09.1998 DE 19841342
(71) Anmelder: MERCK PATENT GmbH, 64293 Darmstadt (DE)
(72) Erfinder: Wenz, Robert, Dr., 61209 Wöllstadt (DE); Pokinskyi, Peter, Dr., 64380 Rossdorf (DE); Ritter, Wolfgang, Dr., 42782 Haan (DE)

(57) **Zusammenfassung**

Die vorliegende Erfindung betrifft verbesserte Reaktivsysteme für die Polymerisation ethylenisch ungesättigter Verbindungen enthaltend als erste Komponente die zur Polymerisation befähigten Monomeren sowie als zweite Komponente ein Härtersystem aus Organo-Borverbindungen, die mit geeigneten Oligomeren stabilisiert sind, wobei diese Komponenten zur Applikation gemischt werden, wobei das Kennzeichen darin liegt, dass den Monomerkomponenten ein organisches Peroxid (Perester, Hydroperoxid, Perether und/oder Peranhydrid) zugemischt wird. Dadurch wird unter anderem eine Erniedrigung der Zeit bis zur Erreichung der Endfestigkeit des Polymers erzielt. Die Reaktivsysteme eignen sich zur Fügung von harten Körpematerialien in menschlichem oder tierischem Gewebe, insbesondere im chirurgischen und/oder zahnmedizinischen Bereich bei der Herstellung von körperresorbierbaren oder körperresistenten Klebern, Zementen und/oder Füllmassen bzw. zur Ausbildung von Kunststoff-Formteilen.

## Beschreibung

Die vorliegende Erfindung beschreibt Reaktivsysteme, die aus ethylenisch ungesättigten Monomeren einerseits und aus sauerstoffreaktiven Organo-Borverbindungen, die mittels Trägermaterialien stabilisiert sind, andererseits aufgebaut sind. Die Monomerkomponente enthält zusätzlich geringe Mengen an Peroxiden.

Die neuen Reaktivsysteme eignen sich prinzipiell für viele Anwendungsgebiete, beispielsweise für den technischen oder handwerklichen Arbeitsbereich, sie haben jedoch eine besondere und bevorzugte Bedeutung für die Anwendung in der Medizin in menschlichem oder tierischem Gewebe. Sie eignen sich als Binde- bzw. Klebemittel für das Verkleben von körpereigenem Hartgewebe wie auch für das Verkleben solchen Gewebes mit Kunststoff und/oder Metall und/oder zur in-situ-Bildung von Kunststoff-Formteilen bei chirurgischen Arbeiten. Insbesondere werden sie auf dem Gebiet der reaktiven Klebstoff- bzw. Zementsysteme des medizinischen und/oder zahnmedizinischen Anwendungsbereiches und im zuletzt genannten Gebiet auch als Füllmassen verwendet.

Sowohl in der Humanmedizin als auch im Bereich der Tiermedizin finden zunehmend Kunststoffe auf Polymerbasis sowie durch Reaktionsauslösung härtbare Reaktivsysteme Bedeutung. Verwiesen sei auf die chirurgischen und/oder zahnmedizinischen Klebstoffe, Zemente, Füllmassen und dergleichen, die gewöhnlich nach ihrer Applikation und Implantation in den lebenden Körper aushärten und dann im Kontakt mit dem lebenden Organismus verbleiben.
Die vorzugsweise in der Praxis eingesetzten härtbaren Klebstoffe bestehen üblicherweise aus den folgenden Komponenten:
- ein oder mehrere radikalisch polymerisierbare, ethylenisch ungesättigte Monomere, die gegebenenfalls in Abmischung mit Inihibitoren gegen eine unerwünschte vorzeitige Reaktionsauslösung vorliegen;
- Ein Startersystem zur Polymerisationsauslösung;
- Polymere zur Kohäsionsverbesserung und zur Einstellung der Viskosität, sowie gegebenenfalls
- aktive Füllstoffe zur Verbesserung der mechanischen Eigenschaften.

Ein häufig verwendetes Startersystem zur Polymerisationsauslösung in ethylenische Doppelbindungen enthaltenden Reaktionssystemen besteht aus sauerstoffreaktiven Organo-Borverbindungen. Dazu gibt es umfangreiche Literatur, daher seien folgende Schriften auch nur als eine kleine Auswahl zitiert: DE 30 41 843, DE 30 41 904, DE 31 43 945, DE 32 01 780, DE 32 04 504 oder auch DE 32 29 635.

In der DE 39 39 164 sind ebenfalls Startersysteme auf Basis von sauerstoffaktiven Organo-Borverbindungen beschrieben, die in Kombination mit einem Trägermaterial vorliegen. Als Träger werden Oligoester von niederen Hydroxycarbonsäuren eingesetzt. Diese Träger verbessern die Stabilität des Systems gegenüber Sauerstoff. Damit so reaktive Borverbindungen wie beispielsweise 9-Borabicyclo[3.3.1]-nonan (9-BBN) überhaupt verarbeitbar und lagerstabil sind, werden sie in eine Matrix eines Esters aus einem mehrwertigen Alkohol und eines Oligomers einer Hydroxycarbonsäure eingebracht.

Aber auch diese Systeme sind nicht optimal, da festgestellt wurde, dass sich die Lagerstabilität umgekehrt proportional zur Konzentration der Borverbindung verhält. Daher wären Startersysteme bzw. Reaktivsysteme wünschenswert, die eine möglichst niedrige Konzentration an Organo-Borverbindungen enthielten, auch wegen der Toxizität der Borverbindungen, ohne dass jedoch diese Reduzierung einen nachteiligen Einfluss auf die Polymerisation hätte.

Die Aufgabe lag daher darin, verbesserte Reaktiv- bzw. Klebstoffsysteme zu finden, die einen möglichst geringen Gehalt an Organo-Borverbindungen, enthalten, und wobei die Kriterien wie Topfzeit, Zeit bis zur Erreichung der Endfestigkeit sowie Festigkeit der Klebung ebenfalls optimiert werden.

Es wurde nun gefunden, dass durch die Zumischung eines Peroxides zur Monomerkomponente des Reaktionssystems die gewünschten Ergebnisse erzielt werden konnten. Als Peroxide kommen hier prinzipiell verschiedene Strukturelemente in Betracht, nämlich sowohl Perester, Hydroperoxide, Perether als auch Peranhydride. Dies ist sehr überraschend, denn es war absolut nicht zu erwarten, dass solche Monomer/Peroxidmischungen handhabbar und lagerstabil sind, da es oftmals schon schwierig ist, die Monomerkomponenten alleine stabil zu lagern, d.h. eine unerwünschte Polymerisation zu verhindern.

Gegenstand der Erfindung ist daher ein Zweikomponenten-Reaktivsystem zur Polymerisation ethylenisch ungesättigter Verbindungen, enthaltend als erste Komponente zur Polymerisation befähigte Monomere (=Reaktivkomponente) sowie als zweite Komponente ein Starter- bzw. Härtersystem aus Organo-Borverbindungen (=Initiatorkomponente), die mit geeigneten Oligomeren stabilisiert sind, wobei diese Komponenten zur Applikation gemischt werden, dadurch gekennzeichnet, dass den Monomeren ein organisches Peroxid zugemischt wird.

Ferner ist Gegenstand der Erfindung die Verwendung der erfindungsgemäß verbesserten Reaktivsysteme auf dem Gebiet der Fügung von harten Körpermaterialien gegebenenfalls zusammen mit Kunststoff und/oder Metall in menschlichem oder tierischem Gewebe, insbesondere auch auf dem Gebiet der reaktiven Klebstoff- bzw. Zementsysteme des chirurgischen und/oder zahnmedizinischen Bereichs.

Das Peroxid wird dabei in die zu polymerisierenden Monomerkomponenten eingebracht, das heißt suspendiert oder darin gelöst. Vorzugsweise werden solche organischen Peroxide verwendet, die sich im Monomersystem lösen.

Das Peroxid steht damit in innigem Kontakt mit den reaktiven Endgruppen der zu polymerisierenden Substanzen, wodurch der Gesamtverlauf der Polymerisation beschleunigt wird. Die Initiierung der Polymerisation erfolgt also einerseits durch die Einwirkung des Starters auf die ethylenisch ungesättigten Verbindungen und andererseits zusätzlich durch das Peroxid, das durch den Starter ebenfalls zum Zerfall gebracht wird und als Polymerisationsbeschleuniger wirkt.

Ein großer Vorteil liegt jedoch auch darin, dass durch Zugabe des Peroxides zum Monomeren der Gehalt an bororganischer Verbindung in der Initiatorkomponente erniedrigt werden kann, was bezüglich der Toxikologie eine wichtige Rolle spielt.

Prinzipiell können alle organischen Peroxide, also Perester, Hydroperoxide, Perether als auch Peranhydride, dafür verwendet werden. Die Auswahl wird jedoch auch bestimmt durch ihre Verteilung in den zu polymerisierenden Substanzen, wobei der Lösung gegenüber Suspensionen der Vorzug gegeben wird. Ferner dürfen die zumischbaren Peroxide oder Reaktionsbeschleuniger nicht schon bei Raumtemperaturen oder unter 50 °C zum Zerfall neigen. Vorzugsweise werden Peroxide verwendet, die eine Halbwertszeit bei 80 °C im Bereich von Stunden bis Tagen aufweisen.

Als besonders bevorzugte Peroxide haben sich *tert*.-Butylperoxybenzoat oder Di-*tert*.-Butylperoxid herausgestellt.

Die Peroxide werden in einer Menge von 0,005 bis 10 Gew.-%, vorzugsweise von 0,1 bis 5 Gew.-%, bezogen auf die Monomerkomponenten, zugemischt.

Als Härter- bzw. Starterkomponente werden in den erfindungsgemäßen Reaktivsystemen Alkyl- und/oder Arylreste aufweisende Borverbindungen oder bicyclische Organo-Borverbindungen eingesetzt, welche mit geeigneten Oligomeren stabilisiert bzw. phlegmatisiert sind.
Eine solche Stabilisierung kann vorzugsweise dadurch erfolgen, dass die entsprechenden organischen Borverbindungen als Gemische mit Oligomeren oder als Additionsprodukte an ungesättigte Fette oder Oligomere infolge einer Hydroborierungsreaktion vorliegen. Diese Startersysteme sind dem Fachmann bekannt und beispielsweise ausführlich beschrieben in den Dokumenten DE 39 39 164, DE 32 07 263 oder DE 32 07 264. Daher soll hier im Folgenden nur kurz darauf eingegangen werden.

Eine bevorzugte Art der Stabilisierung der Organo-Borverbindungen besteht also darin, physikalische Gemische dieser Verbindungen mit Oligomeren herzustellen. Eine Möglichkeit der Phlegmatisierung besteht darin, die organischen Borverbindungen mit einem bei Raumtemperatur flüssigen bis festen, möglichst körperverträglichen organischen Oligomeren bzw. Polymeren homogen zu mischen. Dabei kommen als oligomere bzw. polymere organische Komponenten vorzugsweise bei Raumtemperatur flüssige bis pastös streichbare Oligoester, Oligoamide und/oder Oligoether in Frage.

Ein besonders bevorzugtes Startersystem besteht aus sauerstoffreaktiven Organo-Borverbindungen in Kombination mit einem Trägermaterial, welches als Oligoester von niederen Hydroxycarbonsäuren vorliegt.

Wesentlicher esterbildender Bestandteil dieser Trägermaterialien sind damit Hydroxycarbonsäuren des bevorzugten C-Zahlbereiches von etwa 2 bis 10. Besonders wichtige Hydroxycarbonsäuren sind die Glycolsäure und/oder insbesondere die Milchsäure, die in Form ausgewählter Isomerer oder auch als Isomerengemisch verwendet werden kann. Geeignet sind weiterhin die gegebenenfalls isomeren α- oder β-Hydroxypropionsäuren, α-, β- oder γ-Hydroxybuttersäuren, o-, m- und/oder p-Hydroxybenzoesäure.

Die Oligoester der genannten Hydroxycarbonsäuren können auch unter Mitverwendung von monofunktionellen und/oder mehrfunktionellen Reaktanten hergestellt worden sein, die dazu führen, dass als Träger mit Hydroxylgruppen oder mit Carboxylgruppen terminierte Oligoester der niederen Hydroxycarbonsäuren vorliegen.

Geeignete Co-Reaktanten sind insbesondere Monoalkohole und/oder Monocarbonsäuren, wobei entsprechende Verbindungen mit bis zu 6 C-Atomen bevorzugt sind. Als Trägermaterial besonders geeignet sind allerdings Oligoester, die unter Mitverwendung von mehrwertigen Alkoholen oder auch von mehrfunktionellen Carbonsäuren hergestellt worden sind. Als mehrwertige Alkohole kommen 2- bis 4-funktionelle Alkohole, insbesondere Diole oder auch Glycerin in Betracht. Geeignete mehrfunktionelle Carbonsäuren sind insbesondere Dicarbonsäuren mit 2 bis 10 C-Atomen.

Die Bildung der Oligoester ist eine übliche Veresterungsreaktion, die in Gegenwart geeigneter Katalysatoren in an sich bekannter Weise durchgeführt wird.

Bei beiden Varianten werden die Trägermaterialien bzw. die Oligomeren dann mit den Organo-Borverbindungen zweckmäßigerweise unter Ausschluss von Luftsauerstoff abgemischt, wobei gegebenenfalls unter mäßigem Erwärmen gearbeitet werden kann.

Eine weitere Möglichkeit für ein geeignetes Startersystem besteht darin, Boralkylverbindungen bereitzustellen, die an Fettsäure- und/oder Fettalkoholester oder andere Oligomere gebunden sind. Diese Boralkylverbindungen sind vorzugsweise Umsetzungsprodukte von Borwasserstoff und/oder wenigstens eine B-H-Bindung aufweisenden Organo-Borverbindung mit Oligomeren bzw. Polymeren, die additionsbereite Kohlenstoff-Doppelbindungen enthalten, oder mit Estern olefinisch ungesättigter Fettsäuren und/oder olefinisch ungesättigter Fettalkohole. Die Verbindungen können hergestellt werden, indem man die entsprechenden Ausgangsverbindungen mit additionsbereiten Kohlenstoffdoppelbindungen der Hydroborierung mit Diboran bzw. mono- und/oder disubstituierten Boranen unterwirft.

Besonders bevorzugte Matrixmaterialien können Ester ungesättigter Monocarbonsäuren (ungesättigte Fettsäuren) mit mehrwertigen Alkoholen sein. Als mehrwertige esterbildende Reaktionskomponenten sind insbesondere entsprechende Verbindungen einer Funktionalität bis zu 6, vorzugsweise von 2 bis 4, geeignet. So sind in der bevorzugten Ausführungsform als Matrix für die borhaltigen Substituenten Monocarbonsäuren des angegebenen C-Zahlbereichs mit mehrwertigen Alkoholen - insbesondere mit zweiwertigen, dreiwertigen oder vierwertigen Alkoholen - verestert.

Es kann dabei zweckmäßig sein, dass die polyfunktionelle Esterkomponente eine vergleichsweise niedrige Anzahl von Kohlenstoffatomen besitzt, die beispielsweise im Bereich von 2 bis 10, vorzugsweise im Bereich von 2 bis 6, liegen kann. Als polyfunktionelle Alkohole eignen sich dementsprechend besonders die niederen Glycole wie Ethylenglycol, Propylenglycol-1,2, Propylenglycol-1,3, die C4-Glycole mit endständigen und/oder innenständigen Hydroxylgruppen bzw. entsprechende C5- und C6-Verbindungen. Ein besonders bevorzugter Alkohol ist das Glycerin oder mehrwertige Alkohole von der Art des Pentaerythrits. Umgekehrt können monofunktionelle Fettalkohole mit niederen Polycarbonsäuren, insbesondere niederen Di- oder Tricarbonsäuren, verestert sein.

Allgemein kommen für die beschriebenen Varianten der Starterkomponente als Borverbindungen zahlreiche bekannten Boralkyle in Frage. Typische Vertreter sind beispielsweise 9-Borabicyclo[3.3.1]nonan und dessen Derivate wie B-Methoxy-9-borabicyclo[3.3.1]nonan, Diisopinocampheylboran, Dicyclohexylboran, Thexylboran (2,3-Dimethyl-2-butylboran), 3,5-Dimethylborinan oder auch Diisoamylboran.

Von diesen Verbindungen ist das 9-Borabicyclo[3.3.1]nonan (9-BBN) aus praktischen Gründen ganz besonders bevorzugt.

Eine Zusammenstellung der Herstellungsmöglichkeiten geeigneter Borverbindüngen findet sich in der Monographie Herbert C. Brown, 1975 "Organic Synthesis via Boranes", Verlag John Wiley & Sons.

Der Gehalt an bororganischer Verbindung im Startergemisch kann erfindungsgemäß sehr viel niedriger gewählt werden als es in den bisher bekannten Härtersystemen der Fall ist. Der Gehalt liegt bei 0,5 bis 10 Gew.-%, vorzugsweise im Bereich von 1 bis 5 Gew.-%, wobei sich die Angaben hier auf die Borverbindung beziehen. Dies ist ein bedeutender Vorteil, vor allem auch im Hinblick auf die Toxizität vieler bororganischer Verbindungen.

Der Borgehalt im fertigen Klebstoffgemisch liegt vorzugsweise im Bereich von 0,005 bis 0,2 Gew.-%.

Die polymerisierbare Komponente der erfindungsgemäßen Reaktivsysteme sind Gemische aus einem oder mehreren radikalisch polymerisierbaren Monomeren, Polymeren zur Kohäsionsverbesserung und zur Einstellung der Viskosität, gegebenenfalls aktiven Füllstoffen zur Verbesserung der mechanischen Eigenschaften und gegebenenfalls Stabilisatoren.

Als polymerisierbare Monomere sind neben Methylmethacrylat in Kombination mit Methacrylsäure (Knochenzement) zahlreiche weitere Systeme untersucht worden, von denen einige praktische Bedeutung gewonnen haben, vergleiche hierzu J. M. Antonucci, Polymer Science and Technology 14, 357 (1981).

Besonders geeignet als Monomerkomponenten für den Einsatz im Rahmen dieser Erfindung sind allerdings Oligomerkomponenten, wie sie in den Dokumenten DE 32 05 504 und 32 29 635 beschrieben sind. Es handelt sich hierbei um Bindemittelsysteme für chirurgische Zwecke, die dadurch gekennzeichnet sind, dass sie als resorbierbare (Meth)acrylatkomponenten bei Raumtemperatur flüssige bis feste (Meth)acrylsäureester mit (Meth)acrylatresten an Polyester-Oligomerketten aus Hydroxycarbonsäuren enthalten. Diese radikalisch reaktiven Komponenten können dabei einen oder bevorzugt auch mehrere (Meth)acrylsäurereste an dem Polyester-Oligosegment aufweisen.

Klebstoffsysteme auf Basis solcher Reaktivkomponenten zeichnen sich durch eine Vielzahl von hervorragenden Eigenschaften aus. Insbesondere führen sie zu ausgehärteten Kunststoffen, die vom lebenden Organismus resorbiert werden können.

Das Polyester-Oligosegment wird in einer bevorzugten Ausführungsform aus Monohydroxymonocarbonsäuren gebildet, die eine C-Zahl von etwa 20 im Molekül und insbesondere von etwa 10 nicht überschreiten. Niedere Hydroxycarbonsäuren mit 2 bis 6 C-Atomen können in diesem Zusammenhang besonders wichtig sein. Besonders geeignete Hydroxycarbonsäuren zur Ausbildung dieses Mittelstücks der (Meth)acrylatverbindungen sind Glycolsäure, die isomeren Milchsäuren, die gegebenenfalls isomeren α- oder β-Hydroxypropionsäuren, α-, β- oder γ-Hydroxybuttersäuren, o-, m- und/oder p-Hydroxybenzoesäure. Es können dabei sowohl bestimmte Isomere der genannten Säuren als auch beliebige Gemische zum Einsatz kommen.

Die Polyester-Oligomeren sind dabei zweckmäßigerweise unter Mitverwendung von monofunktionellen und/oder vorzugsweise mehrfunktionellen Reaktanten hergestellt. Als Co-Reaktanten kommen insbesondere Monoalkohole und Monocarbonsäuren in Betracht. Polyfunktionelle Co-Reaktanten bei der Herstellung der Polyester-Oligomeren sind polyfunktionelle Alkohole, insbesondere 2- bis 4-wertige Alkohole oder entsprechende Polycarbonsäuren bzw. ihre funktionellen reaktiven Derivate. Besondere Bedeutung kann hier niederen polyfunktionellen Alkoholen wie Ethylenglycol, Propandiol, insbesondere 1,2-Propandiol, Glycerin und dergleichen zukommen.

In allen geschilderten Fällen entstehen modifizierte Ester-Oligomere, die in an sich bekannter Weise einfach zu den erfindungsgemäß zu verwendenden (Meth)acrylatverbindungen umgesetzt werden können.

In einer ganz besonders bevorzugten Ausführungsform der Erfindung wird als Monomerkomponente ein Produkt eingesetzt, welches vorzugsweise aus Ethylenglycol oder Glycerin, Milchsäure und Glycolsäure aufgebaut ist, wobei das Produkt aus Ethylenglycol und Milchsäure mit einem durchschnittlichen Polymerisationsgrad von kleiner 10 besonders bevorzugt ist.

Des Weiteren sind erfindungsgemäße Klebstoffsysteme besonders bevorzugt, welche insgesamt - also Monomerkomponente und Oligomere zur Stabilisierung der Borverbindung - aus Bausteinen wie Polyethylenglycol und Milchsäure bzw. Glycolsäure aufgebaut sind.

Die Polymere, die aus den Reaktivkomponenten entstehen, können abbauresistent oder körperabbaubar sein. Gleiches gilt für eventuell mitverwendete vorgebildete Polymere. Auch die Startersysteme, die eine Mitverwendung von oligomeren oder polymeren Trägern vorsehen, können wahlweise abbauresistent oder körperresorbierbar ausgebildet sein und in angepasster Weise mit dem Gesamtsystem kombiniert werden.

Werden körperabbaubare Systeme eingesetzt, sind sowohl in der ethylenisch ungesättigten Monomerkomponente wie in den gegebenenfalls zu verwendenden Polymerkomponenten Hydroxycarbonsäurereste, insbesondere entsprechende Reste der Glycolsäure und/oder Milchsäure wesentliche Molekülbausteine.

In vielen Fällen kann es zweckmäßig oder notwendig sein, weitere Hilfsstoffe wie Füllstoffe, beispielsweise Quarzmehl oder dergleichen zuzugeben. Schließlich kann es zweckmäßig sein, mit geeigneten Farbstoffen bzw. Pigmenten einzufärben.

Die Mischungsverhältnisse des Startersystems zu der Reaktivkomponente liegen im üblichen Bereich. Beispielsweise können die Startersysteme im Mengen von etwa 0,5 bis 30 Gew.-% - bezogen auf den zu polymerisierenden Anteil - verwendet werden. Hierbei sei jedoch nochmals erwähnt, dass innerhalb des Startersystems der Gehalt an bororganischer Verbindung erfindungsgemäß wesentlich erniedrigt ist im Vergleich zum Stand der Technik.

Die hier beschriebenen Zweikomponenten-Reaktivsysteme werden zur Fügung - also zum Verkleben - von harten Körpermaterialien, beispielsweise Knochen miteinander, aber auch mit fremden Materialien wie Kunststoff und/oder Metall, in menschlichem oder tierischem Gewebe verwendet. Die Anwendung als Klebstoffe bzw. Zementsysteme im chirurgischen und/oder zahnmedizinischen Bereich ist dabei bevorzugt.

Weiterhin können die Reaktivsysteme der geschilderten Art zur in-situ-Herstellung von individuell ausgebildeten Formteilen, insbesondere im Zusammenhang mit der Verklebung von körpereigenem Hartgewebe, gegebenenfalls zusammen mit Kunststoff und/oder Metall, verwendet werden.

Die Komponenten des Reaktivsystems, also die Monomerkomponenten und die Härter- bzw. Starterkomponenten, werden vorzugsweise nach ihrer - notwendigerweise unter Luftausschluss durchgeführten - Herstellung jeweils steril verpackt, vorzugsweise werden sie in Kunststoffampullen abgefüllt. Die Komponenten werden erst direkt bei der Anwendung zum reaktionsbereiten System vermischt.

Die Lagerung der Monomerkomponenten erfolgt in Ampullen, vorzugsweise in Materialien mit einem hohen Diffusionskoeffizient für Sauerstoff, insbesondere geeignet ist ein low density Polyethylen.

Die Starterkomponente dagegen wird ebenfalls vorzugsweise in einem Kunststoffmaterial gelagert, das für Sauerstoff einen niedrigen Diffusionskoeffizienten hat, oder in einem Material, das mit Metallfolien kaschiert ist.

Vorzugsweise werden die einzelnen Komponenten vor der Abfüllung einer Sterilfiltration unterworfen. Des Weiteren kann es vorteilhaft sein, dass die verschlossenen Ampullen mit Wasserstoffperoxid-Plasma oberflächensterilisiert sind.

Gegenstand der Erfindung ist auch ein Zweikomponenten-Reaktivsystem nach Anspruch 1, welches dadurch gekennzeichnet ist, dass es in Form eines gebrauchsfertigen Sets aus zwei oder mehr getrennten Komponenten vorliegt, dessen eine Komponente die Monomeren und eine andere Komponente die Härterkomponente beinhaltet.

Das Mischen der einzelnen Komponenten kann vorzugsweise mit an sich bekannten Mischsystemen aus der (Medizin)-Technik geschehen, wie z.B. Systemen zur Mischung von Knochenzementen, reaktiven Klebstoffen oder Abformmassen.

Vorzugsweise wird die Mischung durch eine Zweikomponentenspritze vorgenommen. Als Beispiel kann die dem Fachmann bekannte Doppel-Kammer-Spritze mit aufgesetztem Statikmischer genannt werden. Bei diesen Mischsystemen lassen sich auch das Mischungsverhältnis der Komponenten einfach einstellen und variieren. Dadurch wird eine exakte Dosierung gewährleistet sowie eine schnelle Mischung auch ungünstiger Monomer/Härterverhältnisse. Geeignet sind beispielsweise auch Mischsysteme der Firma MIXPAC Systems AG.

Durch das erfindungsgemäße Zugeben eines Peroxides zu den Monomerkomponenten erhält man ein leicht handhabbares System mit niedrigem Borgehalt. Ferner zeichnet sich das Reaktivsystem durch eine handhabbare Topfzeit, eine schnelle Härtungsgeschwindigkeit und auch eine verbesserte Endfestigkeit aus.

Die erfindungsgemäßen Klebstoffe zeichnen sich insbesondere dadurch aus, dass sie nach dem Mischen der Komponenten 50 % ihrer Endfestigkeit innerhalb weniger als 20 Minuten erreichen.

Die Festigkeit dieser Klebstoffsysteme kann beispielsweise bestimmt werden, indem man innerhalb der Topfzeit sandgestrahlte und entfettete Eisenbleche verklebt und die Festigkeit im Zugscherversuch misst.

Bei der Verklebung von hartem Gewebe in der Medizin hängt die erzielte Festigkeit zusätzlich stark von der Vorbehandlung des Knochenmaterials und den Lagerbedingungen der gefügten Teile ab. Für die Verwendung im Organismus sind Festigkeitsmessungen auf entfettetem, trockenem Knochengewebe wenig aussagekräftig. Relevanter erscheint die Verklebung von nicht vorbehandelten, feuchten und fetten Knochen und die Festigkeitsmessung an den erhaltenen Prüfkörpern nach Lagern in (Blut)Ringer-Lösung. Unter diesen simulierten in-vivo-Bedingungen erzielten konventionelle Methacrylatklebstoffe und Knochenzemente auf Knochenmaterial Zugfestigkeiten von ca. 60 N cm⁻² (vgl. hierzu G. Giebel et al. Biomed. Techn. 26, (1981) 170).

Die erfindungsgemäßen Klebstoffe sind somit auch dadurch gekennzeichnet, dass die Festigkeit auf Knochen in Blut bzw. (Blut)Ringerlösung im Zugversuch mindestens 0,3 MPa erreicht.

Gegenstand der Erfindung sind auch Reaktionssysteme nach Anspruch 1, die dadurch gekennzeichnet sind, dass sie im Wesentlichen aus den Bausteinen Polyethylenglycol und Milchsäure bzw. Glycolsäure aufgebaut sind. Solche Systeme sind insbesondere zum Aufbau einer abbaubaren Folie geeignet, die bei Operationen das unerwünschte Zusammenwachsen von Organen verhindert.

Auch ohne weitere Ausführungen wird davon ausgegangen, dass ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen und Veröffentlichungen ist durch Bezugnahme in diese Anmeldung eingeführt.

Die folgenden Beispiele sollen die Erfindung näher erläutern.

### Beispiele:

### I) Oligohydroxycarbonsäuren mit Hydroxylendgruppen

### Darstellung aus Lactid und Ethylenglycol

In einer Edelstahlrührapparatur werden L-Lactid S und Ethylenglycol vorgelegt und mit Phosphorsäure versetzt. Unter Stickstoff wird innerhalb von 1 h auf 100 °C erhitzt, 15 min bei dieser Temperatur gehalten und dann im Verlauf von 30 min auf 130 °C hochgeheizt. Man hält 5 h bei dieser Temperatur und füllt dann das Produkt heiß ab.

Die Zusammensetzung und die Oligomereigenschaften sind der Tabelle 1 (Beispiel 1) zu entnehmen.

**Tabelle 1**

| Oligohydroxycarbonsäuren mit Hydroxylendgruppen aus Lactidsäure und Ethylenglycol | | | | | | |
|---|---|---|---|---|---|---|
| **Beispiel** | **Edukte** | | **Phosphorsäure** | **Säurezahl** | **Berechnetes Molgewicht** *G / mol* | **Beschaffenheit** |
| | **L-Lactid S** *mol* | **Ethylenglycol** *mol* | | | | |
| 1 | 2 | 1 | 4,2 mg/g | 7 | 350 | klar viskos, hellgelb |

### II) Oligohydoxycarbonsäuren mit polymerisierbaren Endgruppen

### a) aus Oligohydroxycarbonsäuren mit endständigen Hydroxylgruppen und Methacrylsäure

In einem Dreihalskolben mit Rührer und Wasserabscheider werden die Oligohydroxycarbonsäure mit Hydroxylendgruppen nach Beispiel 1 und 1,75 Äquivalent Methacrylsäure pro Hydroxylgruppe vorgelegt. Gleichzeitig werden 2000 ppm D,L-Tocopherol und 0,2 Äquivalente Methansulfonsäure zugegeben. Das Gemisch wird unter schnellem Rühren und Durchleiten von Luft auf 900 mbar evakuiert und auf 105°C erhitzt. Nach weiterer Reduktion des Drucks auf 500 mbar wird das gebildete Reaktionswasser am Wasserabscheider abgetrennt. Nach 3,5 h Reaktionszeit wird der Druck auf 100 mbar gesenkt. Die Reaktion ist beendet, sobald sich 85 bis 90 % des zu erwarteten Reaktionswassers gebildet haben. Das Reaktionsprodukt wird zum Neutralisieren auf 100 °C eingestellt und mit Calciumhydroxid versetzt. Nach Zugabe von Celite wird 1 h bei 105 °C, 500 mbar und einem Luftdurchsatz von 300 I nachgerührt. Man filtriert die entstandene Suspension heiß und füllt das erhaltene Filtrat in eine PE-Flasche.

### b) aus Oligohydroxycarbonsäuren mit endständigen Hydroxylgruppen und Methacrylsäurechlorid

In einem Dreihalskolben wird die Oligohydroxycarbonsäure mit Hydroxylendgruppen nach Beispiel 1 in Methyl-*tert.*-butylether gelöst. Anschließend werden 2 Äquivalente pro Hydroxylgruppe wasserfreies Kaliumcarbonat darin suspendiert und innerhalb von 30 min eine Lösung von 0,9 Äquivalenten pro Hydroxylgruppe Methacrylsäurechlorid in Methyl-*tert.*-butylether zugetropft. Die Reaktionslösung wird 24 h bei Raumtemp. gerührt und 48 h ohne Rühren stehengelassen. Der farblose Niederschlag wird abfiltriert und zweimal mit Methyl-*tert.*-butylether gewaschen. Die vereinigten organischen Filtrate werden mit Wasser gewaschen und über Magnesiumsulfat getrocknet. Nach Abfiltrieren des Trockenmittels wird dieses noch zweimal mit Methyl-*tert.*-butylether gewaschen. Nach Zugabe von 500 ppm lonol (2,6-Di-*tert.*-butyl-4-methylphenol) wird langsam innerhalb 24 h bei reduziertem Druck das Lösungsmittel entfernt. Das erhaltene Produkt wird in einer PE-Flasche gelagert.

Die Zusammensetzung der Ansätze und die Eigenschaften der polymerisierbaren Oligomeren sind in der Tabelle 2 (Beispiele 2-3) aufgeführt.

**Tabelle 2**

| Oligohydroxycarbonsäuren mit polymerisierbaren Endgruppen | | | | |
|---|---|---|---|---|
| Beispiel | Methode | Edukt aus Beispiel | Ausbeute % | Beschaffenheit |
| 2 | a) | 1 | >90 | homogen, dünnflüssig, gelb |
| 3 | b) | 1 | 80 | homogen, dünnflüssig, hellgelb |

### c) aus Lactid S und hydroxylierten Methacrylsäureestern

In einem Reaktionskolben werden das Methacrylsäurederivat, L-Lactid S, Magnesiumoxid und Ionol eingewogen und unter Rühren und Lufteinleitung innerhalb von 30 min auf 100 °C erhitzt. Man hält 30 min bei dieser Temperatur und heizt dann innerhalb von 1 h auf 180 °C und hält für 3 h. Nach dem Abkühlen auf Raumtemperatur wird der Katalysator abfiltriert.

Die Zusammensetzung und die Oligomereigenschaften sind der Tabelle 3 (Beispiele 4-7) zu entnehmen. Als Methacrylsäurederivate wurden Hydroxy-ethylmethacrylat (A) sowie Glycerin-dimethacrylat (B) verwendet.

**Tabelle 3**

| Methacrylsäurederivate, verestert mit Oligohydroxycarbonsäuren | | | | | |
|---|---|---|---|---|---|
| **Beispiel** | **Edukte** | | **Magnesiumoxid** | **Berechnetes Molgewicht** g/mol | **Beschaffenheit** |
| | *L-Lactid S* mol | *Methacrylsäure-Derivat* Art/mol | | | |
| 4 | 2 | A/1 | 40mg/g | 418 | klar,viskos |
| 5 | 3 | A/1 | 40 mg/g | 562 | klar, hochviskos |
| 6 | 4 | A/1 | 40 mg/g | 707 | klar, hochviskos |
| 7 | 3 | B/1 | 40 mg/g | 516 | klar, hochviskos |

### III Initiatorkomponente

In einen Rundkolben mit Abziehbogen werden 100 g der Oligohydroxycarbonsäure mit Hydroxylendgruppen nach Beispiel 1 eingefüllt und bei 75 °C unter Rühren für 2 h entgast. Anschließend lässt man über 30 min eine Boran-Lösung zutropfen. Nach Beendigung der Gasentwicklung destilliert man anfangs bei 10 mbar, zur vollständigen Entfernung bei 0,1 mbar das Lösungsmittel ab.

Die Zusammensetzungen der Ansätze sind in der Tabelle 4 (Beispiele 8-11) aufgeführt.

**Tabelle 4**

| Initiatorkomponente | | | |
|---|---|---|---|
| Beispiel | Boran-Lösung | | Beschaffenheit |
| | ml | Art | |
| 8 | 16,4 | 9-BBN, 0,5 M in THF | Homogen, viskos, farblos |
| 9 | 49,2 | 9-BBN, 0,5 M in THF | Homogen, viskos, farblos |
| 10 | 131,2 | 9-BBN, 0,5 M in THF | Homogen, viskos, hellgelb |
| 11 | 25 | BM-9-BBN, 1 M in n-Hexan | Homogen, viskos, farblos |
| 9-BBN = 9-Borabicyclo[3.3.1]nonan BM-9-BBN = B-Methoxy-9-Borabicyclo[3.3.1]nonan | | | |

### IV Reaktivkomponente A

Die in Beispiel 2 und 3 hergestellten Oligohydroxycarbonsäuren mit polymerisierbaren Endgruppen werden unter Rühren durch Zutropfen frisch destillierter Methacrylsäure auf einen Gehalt von 5 % eingestellt. Anschließend werden in diese Monomere die in der Tabelle 5 beschriebenen Mengen Peroxid eingemischt. Die Zusammensetzungen der Ansätze sind in der Tabelle 5 (Beispiele 12-16) aufgeführt.

**Tabelle 5**

| Reaktivkomponente A | | | |
|---|---|---|---|
| **Beispiel** | **Peroxid** | | **Beschaffenheit** |
| | *mg* | *Art* | |
| 12 | 100 | TBPB | Homogen, viskos, hellgelb |
| 13 | 1000 | TBPB | Homogen, viskos, hellgelb |
| 14 | 2000 | TBPB | Homogen, viskos, hellgelb |
| 15 | 1000 | DTBP | Homogen, viskos, hellgelb |
| 16 | 2000 | DTBP | Homogen, viskos, hellgelb |
| DTBP = Di-*tert*.butyl-peroxid TBPB = *tert*.-Butylperoxibenzoat | | | |

Folgende Versuche, Mischungen mit Peroxid herzustellen, führten nicht zu homogenen Mischungen.
(Tabelle 6, Beispiel 17-19)

**Tabelle 6**

| Zumischversuche | | | |
|---|---|---|---|
| **Beispiel** | **Peroxid** | | |
| | *mg* | *Art* | |
| 17 | 100 | BP | keine Einmischung |
| 18 | 2000 | LP | keine Einmischung |
| 19 | 1000 | TBHP | Einmischprobleme: H₂O-Gehalt des Peroxids |
| BP = Dibenzoyl-peroxid LP = Dilauroyl-peroxid TBHP = *tert*.Butyl-hydroperoxid | | | |

### V Reaktivkomponente B

Die in Beispiel 4 und 5 hergestellten, mit Oligohydroxycarbonsäuren veresterten Methacrylsäurederivate werden unter Rühren durch Zutropfen von Triethylenglycol-dimethacrylat (A), Glycerin-dimethacrylat (B) oder dem in Beispiel 7 hergestellten Methacrylsäurederivat (C) und anschließendem Einmischen organischer Peroxiverbindungen zu den Reaktivkomponenten umgesetzt.

Die Zusammensetzungen der Ansätze sind in der Tabelle 7 (Beispiele 20-26) aufgeführt.

**Tabelle 7**

| Reaktivkomponente B | | | | |
|---|---|---|---|---|
| **Beispiel** | **Edukt aus** *Beispiel* | **Zumischung** *Art/Gew.-%* | **Peroxid** *Gen.-%* | **Beschaffenheit** |
| 20 | 4 | - | 1 % | homogen, viskos |
| 21 | 4 | A/10 % | | homogen, viskos |
| 22 | 4 | A/10 % | 2 % | homogen,viskos |
| 23 | 5 | A/20 % | 2 % | homogen, viskos |
| 24 | 5 | A/40 % | 2 % | homogen, viskos |
| 25 | 5 | B/20 % | 4 % | homogen, viskos |
| 26 | 5 | C/50 % | 4 % | homogen, viskos |

### VI Zugscherversuche an geklebten Eisenblechen und Spongiosawürfeln

a) Mit den dargestellten Makromeren wurden an einseitig sandgestrahlten und entfetteten Eisenblechen (analog DIN 53 281 / 53 283) Verklebungen durchgeführt. Die Proben weisen Topfzeiten zwischen zwei und zehn Sekunden auf und werden nach einer Fügezeit von 6 h bei Raumtemperatur auf ihre Zugscherfestigkeit überprüft.
b) Ebenso wurden an Spongiosawürfeln Verklebungen durchgeführt. Die Proben werden nach einer Klebezeit von 5 h bei 37 °C in Phosphatpuffer auf ihre Zugscherfestigkeit überprüft.

1. Geräte: Instron Prüfmaschine Typ 4502, bestehend aus Prüfrahmen mit Traverse, zwei Spannzangen zur Aufnahme der verwendeten Eisenbleche, sowie 10 kN-Meßdose. Software: Instron Serie IX, Programm 03, Zuggeschwindigkeit: 2 mm/min.
2. Prüfkörper:
   a)Stahlbleche 25 x 100 x 1,5 mm, einseitig sandgestrahlt
   b) Spogiosawürfel 2 x 2 x 2 cm, aufgetaut über Nacht in 37 °C- warmem Puffer pH = 7.4, vor der Verklebung 5 min Lagerung in Pferdeserum bei 37 °C.
3. Probenvorbereitung: a) Die Stahlbleche sind vor dem Kleben zu entfetten. Dies geschieht durch zweimalige 15-minütige Behandlung der Teile in Dichlormethan im Ultraschallbad und anschließendes Abreiben von anhaftenden Metallteilen durch ein acetongetränktes Papiertuch.
4. Kleben:
   a) Mittels einer Vorrichtung werden zwei Stahlplättchen so zusammengefügt, dass sie sich um 1 cm überlappen (250 mm² Klebefläche). Ein Stahlplättchen wird auf der Vorrichtung fixiert, der Kleber nach Entlüften der Mischkanüle aufgetragen, das zweite Stahlplättchen aufgelegt und die Klebestelle mit einem Gewicht von 5 kg für 5 min belastet. Die verklebten Stahlplättchen werden aus der Vorrichtung entnommen und bis zur Wertermittlung an der Instron bei Raumtemperatur gelagert.
   b) Die aus dem Pferdeserum entnommenen Spongiosawürfel werden mit einem Einmaltuch abgetupft, Kleber nach Entlüften der Mischkanüle auf den Spongiosawürfel aufgetragen, der zweite Spongiosawürfel aufgesetzt und beide Würfel in einem Schraubstock für 2 min fixiert. Danach wird das nun verklebte Würfelpaar der Vorrichtung entnommen und bis zur Wertermittlung bei 37°C in Phosphatpuffer gelagert.
5. Normen: DIN 53283 Prüfung von Metallklebstoffen, Bestimmung der Klebefestigkeit von einschnittig überlappenden Klebungen. (Zugscherversuch) Die Zusammensetzung der Ansätze und die erzielten Zugscher-Festigkeiten sind in der Tabelle 8 (Beispiele 27-54) aufgeführt.

**Tabelle 8**

| Zugscherfestigkeiten (ZSF) bei der Verklebung von Eisenblechen mit Monomerklebstoffen auf Basis von Oligohydroxycarbonsäuren mit polymerisierbaren Endgruppen sowie Oligohydroxycarbonsäure-veresterten Methacrylsäurederivaten | | | | | |
|---|---|---|---|---|---|
| **Beispiel** | **Initiator- komponente** | **Makromer** | **Mischungsverhältnis** | **Endfestigkeit [N mm**^{**-2**}**]** | |
| | | | | **Blech** | **Spongiosa** |
| | *Beispiel* | *Beispiel* | | | |
| 27 | 10 | - | 1:10 | 8,79 | 0.15-0,21 |
| 28 | 10 | 12 | 1:10 | stark exotherm | |
| 29 | 10 | 13 | 1:10 | stark exotherm | |
| 30 | 10 | 14 | 1:10 | stark exotherm | |
| 31 | 10 | 15 | 1:10 | 11,32 | 0,10-0,28 |
| 32 | 10 | 16 | 1:10 | 11,08 | 0,37 |
| 33 | 10 | - | 1:10 | 3,43 | 0,15 |
| 34 | 8 | - | 1:10 | 4,41 | |
| 35 | 8 | 13 | 1:10 | 11,58 | 0,19 |
| 36 | 8 | 14 | 1:10 | 10,88 | 0,15 |
| 37 | 8 | 15 | 1:10 | 8,79 | 0,18 |
| 38 | 8 | 16 | 1:10 | 7,88 | 0,15 |
| 39 | 8 | 13 | 1:4 | 11,40 | |
| 40 | 9 | - | 1:10 | 4,85 | |
| 41 | 9 | 13 | 1:10 | 10,88 | 0,34 |
| 42 | 9 | 14 | 1:10 | 10,75 | 0,37 |
| 43 | 9 | 15 | 1:10 | 7,52 | |
| 44 | 9 | 16 | 1:10 | 8,54 | |
| 45 | 9 | - | 1:4 | | |
| 46 | 11 | 14 | 1:10 | 10,89 | |
| 47 | 10 | 20 | 1:10 | 7,06 | |
| 48 | 10 | 21 | 1:10 | 6,96 | |
| 49 | 10 | 22 | 1:10 | 7,90 | 0,16 |
| 50 | 10 | 23 | 1:10 | 7,79 | |
| 51 | 10 | 24 | 1:10 | 8,48 | |
| 52 | 10 | 21 | 1:4 | 5,33 | |
| 53 | 10 | 25 | 1:10 | 4,98 | 0,26 |
| 54 | 10 | 26 | 1:10 | 5,33 | 0,37 |

## Patentansprüche

1. Zweikomponenten-Reaktivsystem zur Polymerisation ethylenisch ungesättigter Verbindungen enthaltend als erste Komponente (= Reaktivkomponente) zur Polymerisation befähigte Monomere sowie als zweite Komponente ein Härtersystem aus Organo-Borverbindungen, das mit geeigneten Oligomeren stabilisiert ist (= Initiatorkomponente), wobei beide Komponenten zur Applikation gemischt werden, dadurch gekennzeichnet, dass den Monomeren ein organisches Peroxid, welches aus der Gruppe der Perester, Hydroperoxide, Perether und/oder Peranhydride ausgewählt werden kann, zugemischt wird.

2. Reaktivsystem nach Anspruch 1, dadurch gekennzeichnet, dass die Monomerkomponenten Oligohydroxycarbonsäureacrylate oder -methacrylate sind.

3. Reaktivsystem nach Anspruch 2, dadurch gekennzeichnet, dass die oligomeren Ester unter Mitverwendung von monofunktionellen und/oder difunktionellen Alkoholen oder Carbonsäuren bzw. Carbonsäureanhydriden hergestellt wurden.

4. Reaktivsystem nach Anspruch 3, dadurch gekennzeichnet, dass die Monomerkomponenten aus Ethylenglycol oder Glycerin, Milchsäure und Glycolsäure aufgebaut sind.

5. Reaktivsystem nach mindestens einem der Ansprüche 1 bis 4, dadurch gekennzeichnet, dass das Peroxid in den Monomeren gelöst wird.

6. Reaktivsystem nach mindestens einem der Ansprüche 1 bis 5, dadurch gekennzeichnet, dass die Peroxide eine Halbwertszeit bei 80 °C im Bereich von Stunden bis Tagen aufweisen.

7. Reaktivsystem nach mindestens einem der Ansprüche 1 bis 6, dadurch gekennzeichnet, dass der Gehalt an Peroxid zwischen 0,005 und 10 Gew.-%, vorzugsweise zwischen 0,1 und 5 Gew.-%, bezogen auf die Menge der Monomeren, liegt.

8. Reaktivsystem nach mindestens einem der Ansprüche 1 bis 7, dadurch gekennzeichnet, dass als Peroxide *tert*.-Butylperoxibenzoat oder Di-*tert*.-Benzoylperoxid verwendet werden.

9. Reaktivsystem nach mindestens einem der Ansprüche 1 bis 8, dadurch gekennzeichnet, dass als Härterkomponente Alkyl- und/oder Arylreste aufweisende Borverbindungen oder bicyclische Organo-Borverbindungen eingesetzt werden, welche als Gemische mit Oligomeren oder als Additionsprodukte an ungesättigte Fette oder Oligomere infolge einer Hydroborierungsreaktion vorliegen.

10. Reaktivsystem nach Anspruch 9, dadurch gekennzeichnet, dass die Oligomeren Oligoester von niederen Hydroxycarbonsäuren mit 2 bis 10 C-Atomen sind.

11. Reaktivsystem nach Anspruch 9 oder 10, dadurch gekennzeichnet, dass als Organo-Borverbindung 9-Borabicyclo-[3.3.1]-nonan verwendet wird.

12. Reaktivsystem nach mindestens einem der Ansprüche 9 bis 11, dadurch gekennzeichnet, dass der Gehalt an Borverbindung im Bereich von 0,5 bis 10 Gew.-%, vorzugsweise von 1 bis 5 Gew.-%, bezogen auf die Härterkomponenten, liegt.

13. Klebstoffsystem nach Anspruch 1, dadurch gekennzeichnet, dass der Borgehalt im fertigen Klebstoffgemisch im Bereich von 0,005 bis 0,2 Gew.-% liegt.

14. Klebstoffsystem nach Anspruch 1, dadurch gekennzeichnet, dass es 50 % seiner Endfestigkeit in weniger als 20 Minuten erreicht.

15. Klebstoffe nach Anspruch 1, dadurch gekennzeichnet, dass die Festigkeit auf Knochen in Blut bzw. Blutringerlösung im Zugversuch mindestens 0,3 MPa erreicht.

16. Verwendung der Zweikomponenten-Reaktivsysteme nach Anspruch 1 zur Fügung von harten Körpermaterialien gegebenenfalls zusammen mit Kunststoff und/oder Metall in menschlichem oder tierischem Gewebe.

17. Verwendung nach Anspruch 16, gekennzeichnet durch die Anwendung auf dem Gebiet der reaktiven Klebstoff- bzw. Zementsysteme des chirurgischen und/oder zahnmedizinischen Bereichs.

18. Zweikomponenten-Reaktivsystem nach Anspruch 1, dadurch gekennzeichnet, dass es in Form eines gebrauchsfertigen Sets aus zwei oder mehr getrennten Komponenten vorliegt, dessen eine Komponente die Reaktivkomponenten und eine andere Komponente die Härterkomponente (=Initiatorkomponente) beinhaltet.

19. Reaktivsystem nach Anspruch 18, dadurch gekennzeichnet, dass beide Komponenten über eine Zweikammerspritze mit Statikmischer appliziert werden.

20. Reaktivsystem nach Anspruch 18, dadurch gekennzeichnet, dass die Komponenten nach der Herstellung in Kunststoffampullen abgefüllt werden.

21. Kunststoffampullen nach Anspruch 20, dadurch gekennzeichnet, dass sich für die Lagerung der Reaktivkomponente vorzugsweise Materialien mit hohem Diffusionskoeffizient für Sauerstoff eignen, insbesondere low density Polyethylen (LDPE).

22. Kunststoffampullen nach Anspruch 20, dadurch gekennzeichnet, dass die Initiatorkomponente in einem Kunststoffmaterial gelagert wird, das für Sauerstoff einen niedrigen Diffusionskoeffizienten aufweist oder in Material gelagert wird, das mit Metallfolien kaschiert ist.

23. Reaktivsystem nach Anspruch 18, dadurch gekennzeichnet, dass die Einzelkomponenten getrennt steril hergestellt und verpackt und bei Bedarf zum gebrauchsfertigen Klebstoffgemisch kombiniert werden.

24. Reaktivsystem nach Anspruch 1, dadurch gekennzeichnet, dass es im Wesentlichen aus den Bausteinen Polyethylenglycol und Milchsäure oder Glycolsäure aufgebaut ist.

25. Reaktivsystem nach Anspruch 24, dadurch gekennzeichnet, dass es zum Aufbau einer abbaubaren Folie geeignet ist, die bei Operationen das unerwünschte Zusammenwachsen von Organen verhindert.
